# EUROPÄISCHE PATENTSCHRIFT

(11) **EP 3 127 566 B1**
(45) Veröffentlichungstag und Bekanntmachung des Hinweises auf die Patenterteilung: **31.01.2018**
(21) Anmeldenummer: 16182113.7
(22) Anmeldetag: 30.07.2016
(51) Int. Cl.: A61M 5/172, A61M 5/168, A61M 5/142

(54) **IMPLANTIERBARE INFUSIONSPUMPE MIT FÜLLSTANDSMESSER**
IMPLANTABLE INFUSION PUMP WITH FILL LEVEL SENSOR
POMPE À PERFUSION IMPLANTABLE À JAUGE

(30) Priorität: 03.08.2015 DE 102015010063
(43) Veröffentlichungstag der Anmeldung: 08.02.2017
(73) Patentinhaber: Tricumed Medizintechnik GmbH, 24143 Kiel (DE)
(72) Erfinder: Tönnies, Jan Gerrit, 24105 Kiel (DE); Otto, Karl-Heinz, 24146 Kiel (DE)
(74) Vertreter: Lobemeier, Martin Landolf

(56) Entgegenhaltungen:
- US-A1- 2004 073 196
- US-A1- 2005 075 624
- US-A1- 2008 306 466
- US-A1- 2010 217 244
- US-A1- 2015 209 513

## Beschreibung

Die Erfindung betrifft eine implantierbare Einheit zur Überwachung des Katheters einer implantierten Infusionspumpe auf Durchgängigkeit.

Implantierbare Infusionspumpen sind beispielsweise aus der DE 195 14 237 A1 und der DE 196 24 215 C1 bekannt. US2005/0075624 offenbart einen implantierbaren Drucksensor zum Überwachen eines Katheters, gekoppelt mit einer Kontrolleinheit.

Für einige Anwendungsfälle ist es von Bedeutung, dass eine unterunterbrochene Versorgung des Patienten sichergestellt ist. Schon relativ kurzzeitige Unterbrechungen der Medikamentengabe können für diesen fatale Konsequenzen haben. Ein derartiges Ereignis droht, wenn der zu dem Applikationsort im Patienten führende Katheter abknickt, abgedrückt wird oder durch sich an dessen Austrittsende ansammelnde Blutbestandteile "zuwächst".

Es ist daher bekannt, in der Infusionspumpe Drucksensoren anzuordnen, die den Nichtabfluss des von dem Katheter abzugebenden Medikament erkennen und dann einen ein akustisches oder ein Vibrationssignal abgebenden Signalgeber ansteuern, der den Patienten auf das Ereignis aufmerksam macht.

Der Erfindung liegt die Aufgabe zugrunde, eine Katheterüberwachung bei bereits implantierter, nicht mit einer solchen Einrichtung versehenen Infusionspumpe zu ermöglichen.

Diese Aufgabe wird erfindungsgemäß durch die implantierbare Einheit mit den Merkmalen von Anspruch 1 gelöst. Der Unteranspruch gibt eine vorteilhafte Ausgestaltung der Erfindung wieder.

Erfindungsgemäß ist also eine implantierbare Einheit zur Überwachung des Katheters einer implantierten Infusionspumpe auf Durchgängigkeit vorgesehen, mit einem eine Kammer ausbildenden Gehäuse, einem mit der Infusionspumpe kommunizierenden Kammerzufluss, einem mit dem Katheter kommunizierenden Kammerabfluss, einer Spannungsquelle, einem auf einen vorgegebenen ersten Wert übersteigenden Druck in der Kammer ansprechenden ersten Schalter und einem beim Schließen des Schalters ansprechenden akustischen Signalgeber.

Bevorzugt ist ein auf einen vorgegebenen zweiten Wert unterschreitenden Druck in der Kammer ansprechender zweiter Schalter vorgesehen.

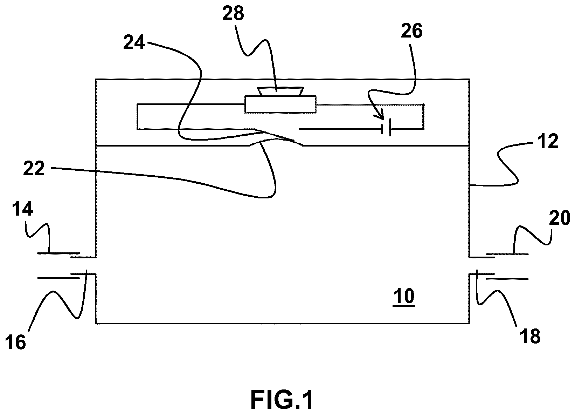

Die Erfindung wird im Folgenden anhand einer Zeichnung erläutert, deren einzige Figur eine schematische Schnittdarstellung der Einheit darstellt.

Die Einheit besteht aus einem eine Kammer 10 ausbildenden Gehäuse 12, das mit einem über ein Schlauchstück 14 mit dem Ausgang der Infusionspumpe verbunden Zufluss 16 und einem Abfluss 18, an den das zum Applikationsort in dem Patienten führende Katheter 20 angeschlossen ist, versehen ist. Die von dem dauerhaft abzugebenden Medikament durchflossene Kammer 10 wirkt (hier über eine Membran 22) auf einen Schalter 24, der einen Stromkreis, in dem eine Spannungsquelle 26 und ein akustischer Signalgeber 28 liegen, bei Überschreiten eines vorgegebenen Drucks, der erheblich über dem liegt, der bei durchgängigem Katheter 20 erreicht wird, schließt. Bei einem Verschluss des Katheters 20 wird der Druck in der Kammer 10 auf den Druck des nachströmenden Medikaments ansteigen, die Membran 22 wird sich durchbiegen, der Schalter 24 schließen und der Signalgeber 28 ansprechen.

Bei einer bevorzugten, zeichnerisch nicht dargestellten Ausführungsform ist ein zu dem ersten Schalter parallel liegender zweiter Schalter vorgesehen, der den Stromkreis schließt, wenn der Kammerdruck einen vorgegebenen zweiten Wert unterschreitet und damit das Fehlen eines Zuflusses des Medikaments anzeigt. Bei dieser Ausführungsform kann es sinnvoll sein, vor dem Abfluss einen Restriktor mit einem (gegenüber dem in der Infusionspumpe) relativ geringen Restriktionswert vorzusehen.

## Patentansprüche

1. Implantierbare Einheit zur Überwachung des Katheters (10) einer implantierten Infusionspumpe auf Durchgängigkeit,
**gekennzeichnet durch**
- ein eine Kammer (10) ausbildendes Gehäuse (12), wobei die Kammer (10)
∘ einen mit der Infusionspumpe kommunizierenden Kammerzufluss (16),
∘ einen mit dem Katheter (20) kommunizierenden Kammerabfluss (18), und
∘ eine bei Verschluss des Katheters (20) und aufgrund eines infolge von der Infusionspumpe nachfolgenden Medikaments in der Kammer (10) ansteigenden Drucks durchbiegende Membran (22)
aufweist, und
- einen Stromkreis mit
∘ einer Spannungsquelle (26),
∘ einem Schalter (24) und
∘ einen beim Schließen des Schalters (24) ansprechenden akustischen Signalgeber (28),
wobei die sich bei Überschreiten eines vorgegebenen Drucks, der erheblich über dem liegt, der bei durchgängigem Katheter erreicht wird, durchbiegende Membran auf den Schalter wirkt und diesen schließt.

## Claims

1. An implantable unit for monitoring the catheter (10) of an implanted infusion pump for patency,
**characterized by**
- a housing (12) forming a chamber (10), wherein the chamber (10) has
∘ a chamber inlet (16) communicating with the infusion pump,
∘ a chamber outlet (18) communicating with the catheter (20), and
∘ a membrane (22) that flexes in the event of blockage of the catheter (20) and due to a rising pressure in the chamber (10) as a consequence of medicament flowing in from the infusion pump,
and
- a power circuit comprising
∘ a voltage source (26),
∘ a switch (24), and
∘ an acoustic signal generator (28) which reacts when the switch (24) closes,
wherein the membrane, flexing when a predetermined pressure that is substantially higher than the pressure reached with a patent catheter is exceeded, acts on the switch and closes it.

## Revendications

1. Unité implantable pour surveiller le débit du cathéter (10) d'une pompe à perfusion implantée,
**caractérisée par**
- un boîtier (12) formant une chambre (10), la chambre (10) présentant
∘ une amenée (16) communiquant avec la pompe à perfusion,
∘ une évacuation (18) communiquant avec le cathéter (20), et
∘ une membrane (22) pliant lors de la fermeture du cathéter et en raison de l'augmentation de la pression dans la chambre à la suite du passage d'un médicament dans la pompe à perfusion
et
- un circuit électrique comportant
∘ une source de tension (26),
∘ un commutateur (24) et
∘ un émetteur de signaux sonores (28) réagissant à la fermeture du commutateur (24),
la membrane pliant lors du dépassement d'une pression prédéfinie, qui est nettement supérieure à la pression atteinte lorsque le cathéter peut être traversé, agissant sur le commutateur pour le fermer.
